# EUROPÄISCHE PATENTSCHRIFT

(11) **EP 0 994 904 B1**
(45) Veröffentlichungstag und Bekanntmachung des Hinweises auf die Patenterteilung: **12.11.2003**
(21) Anmeldenummer: 98951374.2
(22) Anmeldetag: 10.09.1998
(51) Int. Cl.: C07K 16/12, C12N 15/13, C12P 21/00, A61K 39/40

(54) **AMINOSÄURESEQUENZEN ZUR THERAPIE UND PROPHYLAXE VON ERKRANKUNGEN DURCH (CLOSTRIDIUM DIFFICILE) TOXINE**
AMINO ACID SEQUENCES FOR THERAPEUTICAL AND PROPHYLACTIC APPLICATIONS TO DISEASES DUE TO CLOSTRIDIUM DIFFICILE TOXINS
SEQUENCES D'ACIDES AMINES A USAGE THERAPEUTIQUE ET PROPHYLACTIQUE POUR LES MALADIES CAUSEES PAR DES TOXINES DE CLOSTRIDIUM DIFFICILE

(30) Priorität: 10.09.1997 DE 19739685
(43) Veröffentlichungstag der Anmeldung: 26.04.2000
(73) Patentinhaber: Von Eichel-Streiber, Christoph, 55444 Schweppenhausen (DE)
(72) Erfinder: VON EICHEL-STREIBER, Christoph, D-55444 Schweppenhausen (DE); MOOS, Michael, D-55128 Mainz (DE)
(74) Vertreter: Rudolph, Ulrike, Dr.
(86) Internationale Anmeldenummer: EP9805759
(87) Internationale Veröffentlichungsnummer: WO99012971

(56) Entgegenhaltungen:
- EP-A- 0 411 893
- WO-A-91/10737
- WO-A-91/18293
- WO-A-92/07075
- WO-A-96/12802
- US-A- 4 879 218
- US-A- 5 071 759
- LEWIS A P ET AL: "IMMUNOGLOBULIN COMPLEMENTARITY-DETERMINING REGION GRAFTING BY RECOMBINANT POLYMERASE CHAIN REACTION TO GENERATE HUMANISED MONOCLONAL ANTIBODIES" GENE, Bd. 101, Nr. 2, 1. Januar 1991, Seiten 297-302, XP000310577
- LYERLY ET AL: "Vaccination against lethal Clostridium difficile Enterocolitis with a nontoxic recombinant peptide of toxin A" CURRENT MICROBIOLOGY, Bd. 21, Juli 1990, Seiten 29-32, XP002083271
- CORTHIER G ET AL: "PROTECTION AGAINST EXPERIMENTAL PSEUDOMEMBRANOUS COLITIS IN GNOTOBIOTIC MICE BY USE OF MONOCLONAL ANTIBODIES AGAINST CLOSTRIDIUM DIFFICILE TOXIN A" INFECTION AND IMMUNITY, Bd. 59, Nr. 3, März 1991, Seiten 1192-1195, XP002912253
- C.A. KETTLEBOROUGH ET AL.,: "Optimization of primers for cloning libraries of mouse immunoglobulin genes using the polymerase chain reaction" EUROPEAN JOURNAL OF IMMUNOLOGY, Bd. 23, 1993, Seiten 206-211, XP000573168
- L.A. BARROSO ET AL., : "Mutagenesis of the Clostridium difficile toxin B gene and effect on cytotoxic activity" MICROBIAL PATHOGENESIS, Bd. 16, Nr. 4, 1994, Seiten 297-303, XP002100213
- KINK J A ET AL: "ANTIBODIES TO RECOMBINANT CLOSTRIDIUM DIFFICILE TOXINS A AND B ARE EFFECTIVE TREATMENT AND PREVENT RELAPSE OF C. DIFFICILE-ASSOCIATED DISEASE IN A HAMSTER MODEL OF INFECTION" INFECTION AND IMMUNITY, Bd. 66, Nr. 5, Mai 1998, Seiten 2018-2025, XP002912254
- WARD ET AL: "Delivery of non-toxic fragments of Clostridium difficile toxin A to the mucosal immune system" REVIEWS IN MEDICAL MICROBIOLOGY, Bd. 8, Nr. SUPPL. 01, Dezember 1997, Seiten S34-S36, XP002083272
- Freer et al. (Eds.), Bacterial Protein Toxins, Zbl. Bakt. Suppl. 24, Gustav Fischer, Stuttgart, Jena, New York, 1994
- FEMS Microbiology Letters, Bd. 155, 01.10.1997, 45 - 54

### SEQUENZPROTOKOLL

(1) ALLGEMEINE ANGABEN:
   (i) ANMELDER:
      (A) NAME: Dr. Christoph von Eichel-Streiber
      (B) STRASSE: Bingerweg 15
      (C) ORT: Schweppenhausen
      (D) BUNDESLAND: Rheinland-Pfalz
      (E) LAND: Bundesrepublik Deutschland
      (F) POSTLEITZAHL: 55444
      (G) TELEFON: 06724/3398
      (H) TELEFAX: 06724/941078
   (ii) BEZEICHNUNG DER ERFINDUNG: Aminosäuresequenzen zur Therapie und Prophylaxe von Erkrankungen durch Clostridium difficile Toxine
   (iii) ANZAHL DER SEQUENZEN: 16
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
   (vi) DATEN DER URANMELDUNG:
      (A) ANMELDENUMMER: DE 19739685.2
      (B) ANMELDETAG: 10-SEP-1997
(2) ANGABEN ZU SEQ ID NO: 1:
   (i) SEQUENZKENNZEICHEN:
      (A) LÄNGE: 15 Basenpaare
      (B) ART: Nucleotid
      (C) STRANGFORM: Einzelstrang
      (D) TOPOLOGIE: nicht bekannt
   (ii) ART DES MOLEKÜLS: cDNA
   (iii) HYPOTHETISCH: NEIN
   (iv) ANTISENSE: NEIN
   (v) ART DES FRAGMENTS: schwere Kette CDR1
   (vi) URSPRÜNGLICHE HERKUNFT:
      (B) STAMM: Monoclonal antibody TTC8
      (H) ZELLLINIE: Hybridoma
   (vii) UNMITTELBARE HERKUNFT:
      (A) BIBLIOTHEK: DSM ACC 2322
   (ix) MERKMAL:
      (A) NAME/SCHLÜSSEL: CDS
      (B) LAGE:1..15
   (xi) SEQUENZBESCHREIBUNG: SEQ ID NO: 1:
   AAC TAC TGG ATG AAC
   Asn Tyr Trp Met Asn
     1               5
(2) ANGABEN ZU SEQ ID NO: 2:
   (i) SEQUENZKENNZEICHEN:
      (A) LÄNGE: 5 Aminosäuren
      (B) ART: Aminosäure
      (D) TOPOLOGIE: linear
   (ii) ART DES MOLEKÜLS: CDR1 Proteinsequenz der schweren Kette
   (xi) SEQUENZBESCHREIBUNG: SEQ ID NO: 2:
   Asn Tyr Trp Met Asn
     1               5
(2) ANGABEN ZU SEQ ID NO: 3:
   (i) SEQUENZKENNZEICHEN:
      (A) LÄNGE: 51 Basenpaare
      (B) ART: Nucleotid
      (C) STRANGFORM: Einzelstrang
      (D) TOPOLOGIE: nicht bekannt
   (ii) ART DES MOLEKÜLS: cDNA
   (iii) HYPOTHETISCH: NEIN
   iv) ANTISENSE: NEIN
   (v) ART DES FRAGMENTS: schwere Kette CDR2
   (vi) URSPRÜNGLICHE HERKUNFT:
      (B) STAMM: Monoclonal antibody TTC8
      (H) ZELLLINIE: Hybridoma
   (vii) UNMITTELBARE HERKUNFT:
      (A) BIBLIOTHEK: DSM ACC 2322
   (ix) MERKMAL:
      (A) NAME/SCHLÜSSEL: CDS
      (B) LAGE:1..51
   (xi) SEQUENZBESCHREIBUNG: SEQ ID NO: 3:
   CGG ATT TAT CCT GGA GAT GGA GAT GCT CAC TAC AAT GGG AAG TTC AAG
   Arg Ile Tyr Pro Gly Asp Gly Asp Ala His Tyr Asn Gly Lys Phe Lys
                     5                  10                  15
   GGC
   Gly
(2) ANGABEN ZU SEQ ID NO: 4:
   (i) SEQUENZKENNZEICHEN:
      (A) LÄNGE: 17 Aminosäuren
      (B) ART: Aminosäure
      (D) TOPOLOGIE: linear
   (ii) ART DES MOLEKÜLS: CDR2 - Proteinsequenz der schweren Kette
   (xi) SEQUENZBESCHREIBUNG: SEQ ID NO.4:
   Arg Ile Tyr Pro Gly Asp Gly Asp Ala His Tyr Asn Gly Lys Phe Lys
   1               5                   10                  15
   Gly
(2) ANGABEN ZU SEQ ID NO: 5:
   (i) SEQUENZKENNZEICHEN:
      ( A) LÄNGE: 33 Basenpaare
      (B) ART: Nucleotid
      (C) STRANGFORM: Einzelstrang
      (D) TOPOLOGIE: nicht bekannt
   (ii) ART DES MOLEKÜLS: cDNA
   (iii) HYPOTHETISCH: NEIN
   (v) ART DES FRAGMENTS: schwere Kette CDR3
   (vi) URSPRÜNGLICHE HERKUNFT:
      (B) STAMM: Monoclonal antibody TTC8
      (H) ZELLLINIE: Hydridoma
   (vii) UNMITTELBARE HERKUNFT:
      (A) BIBLIOTHEK: DSM ACC 2322
   (ix) MERKMAL:
      (A) NAME/SCHLÜSSEL: CDS
      (B) LAGE:1..33
   (xi) SEQUENZBESCHREIBUNG: SEQ ID NO.5:
(2) ANGABEN ZU SEQ ID NO.6:
   (i) SEQUENZKENNZEICHEN:
      (A) LÄNGE: 11 Aminosäuren
      (B) ART: Aminosäure
      (D) TOPOLOGIE: linear
   (ii) ART DES MOLEKÜLS: CDR3-Proteinsequenz schwere Kette
   (xi) SEQUENZBESCHREIBUNG: SEQ ID NO.6:
(2) ANGABEN ZU SEQ ID NO.7:
   (i) SEQUENZKENNZEICHEN:
      (A) LÄNGE: 33 Basenpaare
      (B) ART: Nucleotid
      (C) STRANGFORM: Einzelstrang
      (D) TOPOLOGIE: nicht bekannt
   (ii) ART DES MOLEKÜLS: cDNA
   (v) ART DES FRAGMENTS: hypervariable Region (CDR) leichte Kette
   (vi) URSPRÜNGLICHE HERKUNFT:
      (B) STAMM: Monoclonal antibody TTC8
      (H) ZELLLINIE: Hybridoma
   (vii) UNMITTELBARE HERKUNFT:
      (A) BIBLIOTHEK: DSM ACC 2322
   (ix) MERKMAL:
      (A) NAME/SCHLÜSSEL: CDS
      (B) LAGE:1..33
   (xi) SEQUENZBESCHREIBUNG: SEQ ID NO.7:
   AAG GCC AGT CAG AAT GTG GGT ACT AAT GTA GCC
   Lys Ala Ser Gln Asn Val Gly Thr Asn Val Ala
                   5                   10
(2) ANGABEN ZU SEQ ID NO: 8:
   (i) SEQUENZKENNZEICHEN:
      (A) LÄNGE: 11 Aminosäuren
      (B) ART: Aminosäure
      (D) TOPOLOGIE: linear
   (ii) ART DES MOLEKÜLS: CDR1 Proteinsequenz leichte Kette
   (xi) SEQUENZBESCHREIBUNG: SEQ ID NO: 8:
(2) ANGABEN ZU SEQ ID NO: 9:
   (i) SEQUENZKENNZEICHEN:
      (A) LÄNGE: 21 Basenpaare
      (B) ART: Nucleotid
      (C) STRANGFORM: Einzelstrang
      (D) TOPOLOGIE: nicht bekannt
   (ii) ART DES MOLEKÜLS: cDNA
   (iii) HYPOTHETISCH: nein
   (v) ART DES FRAGMENTS: leichte Kette CDR2
   (vi) URSPRÜNGLICHE HERKUNFT:
      (B) STAMM: Monoclonal antibody TTC8
      (H) ZELLLINIE: Hybridoma
   (vii) UNMITTELBARE HERKUNFT:
      (A) BIBLIOTHEK: DSM ACC 2322
   (ix) MERKMAL:
      (A) NAME/SCHLÜSSEL: CDS
      (B) LAGE:1..21
   (xi) SEQUENZBESCHREIBUNG: SEQ ID NO.9:
   TCG CCA TCC TAC CGG TAC AGT
   Ser Pro Ser Tyr Arg Tyr Ser
                       5
(2) ANGABEN ZU SEQ ID NO: 10:
   (i) SEQUENZKENNZEICHEN:
      (A) LÄNGE: 7 Aminosäuren
      (B) ART: Aminosäure
      (D) TOPOLOGIE: linear
   (ii) ART DES MOLEKÜLS: CDR2 Proteinsequenz leichte Kette
   (xi) SEQUENZBESCHREIBUNG: SEQ ID NO: 10:
   Ser Pro Ser Tyr Arg Tyr Ser
    1              5
(2) ANGABEN ZU SEQ ID NO: 11:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: nicht bekannt
   (ii) ART DES MOLEKÜLS: cDNA
   (iii) HYPOTHETISCH: nein
   (iv) ANTISENSE: nein
   (v) ART DES FRAGMENTS: leichte Kette CDR3
   (vi) URSPRÜNGLICHE HERKUNFT:
      (B) STAMM: Monoclonal antibody TTC8
      (H) ZELLLINIE: Hybridoma
   (vii) UNMITTELBARE HERKUNFT:
      (A) BIBLIOTHEK: DSM ACC 2322
   (ix) MERKMAL:
      (A) NAME/SCHLÜSSEL: CDS
      (B) LAGE:1..27
   (xi) SEQUENZBESCHREIBUNG: SEQ ID NO: 11:
   CAG CAA TAT AAT AGC TAT CCT CTT ACG
   Gln Gln Tyr Asn Ser Tyr Pro Leu Thr
                   5
(2) ANGABEN ZU SEQ ID NO: 12:
   (i) SEQUENZKENNZEICHEN:
      (A) LÄNGE: 9 Aminosäuren
      (B) ART: Aminosäure
      (D) TOPOLOGIE: linear
   (ii) ART DES MOLEKÜLS: CDR3 Proteinsequenz leichte Kette
   (xi) SEQUENZBESCHREIBUNG: SEQ ID NO: 12:
   Gln Gln Tyr Asn Ser Tyr Pro Leu Thr
    1               5
(2) ANGABEN ZU SEQ ID NO: 13:
   (i) SEQUENZKENNZEICHEN:

(A) LÄNGE: 341 Basenpaare
(B) ART: Nucleotid
(C) STRANGFORM: Einzelstrang
(D) TOPOLOGIE: nicht bekannt
   (ii) ART DES MOLEKÜLS: cDNA
   (iii) HYPOTHETISCH: NEIN
   (v) ART DES FRAGMENTS: variable Region VH der schweren Kette
   (vi) URSPRÜNGLICHE HERKUNFT:
      (B) STAMM: Monoclonal antibody TTC8 (H) ZELLLINIE: Hybridoma
   (vii) UNMITTELBARE HERKUNFT:
      (A) BIBLIOTHEK: DSM ACC 2322
   (ix) MERKMAL:
      (A) NAME/SCHLÜSSEL: CDS
      (B) LAGE: 1....341
   (IX) MERKMAL:
      (A) NAME/SCHLÜSSEL: primer_bind
      (B) LAGE 1....21
      (D) SONSTIGE ANGABEN: /label=VH5PRIM
   (ix) MERKMAL:
      (A) NAME/SCHLÜSSEL: primer_bind
      (B) LAGE:325..341
      (D) SONSTIGE ANGABEN:/label= VH3PRIM
   (xi) SEQUENZBESCHREIBUNG: SEQ ID NO: 13:
   GTC GAC CTG CAG CAG TCT GGA CCT GAG CTG GTG AAG CCT GGG GCC TCA
   Val Asp Leu Gln Gln Ser Gly Pro Glu Leu Val Lys Pro Gly Ala Ser
    1               5                  10                  15
   GTG AAG ATT TCC TGC AAA GCT TCT GGC TAC GCA TTC AGT AAC TAC TGG
   Val Lys Ile Ser Cys Lys Ala Ser Gly Tyr Ala Phe Ser Asn Tyr Trp
               20                  25                  30
   ATG AAC TGG GTG AAG CAG AGG CCT GGA AAG GGT CTT GAG TGG ATT GGA
   Met Asn Trp Val Lys Gln Arg Pro Gly Lys Gly Leu Glu Trp Ile Gly
           35                  40                  45
   CGG ATT TAT CCT GGA GAT GGA GAT GCT CAC TAC AAT GGG AAG TTC AAG
   Arg Ile Tyr Pro Gly Asp Gly Asp Ala His Tyr Asn Gly Lys Phe Lys
       50                  55                  60
   GGC AAG GCC ACA CTG ACT GCA GAC AAA TCC TCC AGC ACA GCC TAC ATG
   Gly Lys Ala Thr Leu Thr Ala Asp Lys Ser Ser Ser Thr Ala Tyr Met
   65                  70                  75                  80
   CAA CTC AGC AGC CTG ACA TCT GAG GAC TCT GCG GTC TAC TTC TGT GCA
   Gln Leu Ser Ser Leu Thr Ser Glu Asp Ser Ala Val Tyr Phe Cys Ala
                   85                  90                  95
   AGA GGG GGG AAT TAC GAC GAC AGG GTC TTT GAC TAC TGG GGC CAA GGG
   Arg Gly Gly Asn Tyr Asp Asp Arg Val Phe Asp Tyr Trp Gly Gln Gly
               100                 105                 110
   TCG AC 341
   Ser
(2) ANGABEN ZU SEQ ID NO: 14:
   (i) SEQUENZKENNZEICHEN:
      (A) LÄNGE: 113 Aminosäuren
      (B) ART: Aminosäure
      (D) TOPOLOGIE: linear
   (ii) ART DES MOLEKÜLS: Proteinsequenz der variablen Region VH der schweren Kette
   (xi) SEQUENZBESCHREIBUNG: SEQ ID NO: 14:
   Val Asp Leu Gln Gln Ser Gly Pro Glu Leu Val Lys Pro Gly Ala Ser
     1               5                  10                  15
   Val Lys Ile Ser Cys Lys Ala Ser Gly Tyr Ala Phe Ser Asn Tyr Trp
                20                  25                  30
   Met Asn Trp Val Lys Gln Arg Pro Gly Lys Gly Leu Glu Trp Ile Gly
            35                  40                  45
   Arg Ile Tyr Pro Gly Asp Gly Asp Ala His Tyr Asn Gly Lys Phe Lys
        50                  55                  60
   Gly Lys Ala Thr Leu Thr Ala Asp Lys Ser Ser Ser Thr Ala Tyr Met
    65                  70                  75                  80
   Gln Leu Ser Ser Leu Thr Ser Glu Asp Ser Ala Val Tyr Phe Cys Ala
                    85                  90                  95
   Arg Gly Gly Asn Tyr Asp Asp Arg Val Phe Asp Tyr Trp Gly Gln Gly
               100                 105                 110
   Ser
(2) ANGABEN ZU SEQ ID NO: 15:
   (i) SEQUENZKENNZEICHEN:
      (A) LÄNGE: 312 Basenpaare
      (B) ART: Nucleotid
      (C) STRANGFORM: Einzelstrang
      (D) TOPOLOGIE: nicht bekannt
   (ii) ART DES MOLEKÜLS: cDNA
   (iii)HYPOTHETISCH:nein
   (iv) ANTISENSE: NEIN
   (v) ART DES FRAGMENTS: variable Region der leichten Kette
   (vi) URSPRÜNGLICHE HERKUNFT:
      (B) STAMM: Monoclonal antibody TTC8
      (H) ZELLLINIE: Hybridoma
   (vii) UNMITTELBARE HERKUNFT:
      (A) BIBLIOTHEK: DSM ACC 2322
   (ix) MERKMAL:
      (A) NAME/SCHLÜSSEL: primer_bind
      (B) LAGE:1..18
      (D) SONSTIGE ANGABEN:/label= VL5Prim
   (ix) MERKMAL:
      (A) NAME/SCHLÜSSEL: primer_bind
      (B) LAGE:289..312
      (D) SONSTIGE ANGABEN:/label= VL3PRIM
   (ix) MERKMAL:
      (A) NAME/SCHLÜSSEL: CDS
      (B) LAGE:1..312
   (xi) SEQUENZBESCHREIBUNG: SEQ ID NO: 15:
   GAG CTC ACC CAG TCT CCA AAA TTC ATG TCC ACA TCA GTA GGA GAC AGG
   Glu Leu Thr Gln Ser Pro Lys Phe Met Ser Thr Ser Val Gly Asp Arg
    1               5                  10                  15
   GTC AGC GTC ACC TGC AAG GCC AGT CAG AAT GTG GGT ACT AAT GTA GCC
   Val Ser Val Thr Cys Lys Ala Ser Gln Asn Val Gly Thr Asn Val Ala
               20                  25                  30
   TGG TAT CAA CAG AAA CCA GGG CAA TCT CCT AAA ACA CTG ATT TAC TCG
   Trp Tyr Gln Gln Lys Pro Gly Gln Ser Pro Lys Thr Leu Ile Tyr Ser
            35                  40                  45
   CCA TCC TAC CGG TAC AGT GGA GTC CCT GAT CGC TTC ACA GGC AGT GGA
   Pro Ser Tyr Arg Tyr Ser Gly Val Pro Asp Arg Phe Thr Gly Ser Gly
        50                  55                  60
   TCT GGG ACA GAT TTC ACT CTC ACC ATC AGC AAT GTG CAG TCT GTT GAC
   Ser Gly Thr Asp Phe Thr Leu Thr Ile Ser Asn Val Gln Ser Val Asp
   65                  70                  75                  80
   TTG GCA GAG TAT TTC TGT CAG CAA TAT AAT AGT TAT CCT CTT ACG TTC
   Leu Ala Glu Tyr Phe Cys Gln Gln Tyr Asn Ser Tyr Pro Leu Thr Phe
                       85                  90                  95
   GGC TCG GGG ACC AAG CTG GAG CTC
   Gly Ser Gly Thr Lys Leu Glu Leu
               100
(2) ANGABEN ZU SEQ ID NO: 16:
   (i) SEQUENZKENNZEICHEN:
      (A) LÄNGE: 104 Aminosäuren
      (B) ART: Aminosäure
      (D) TOPOLOGIE: linear
   (ii) ART DES MOLEKÜLS: Proteinsequenz der variablen Region VL der leichten Kette
   (xi) SEQUENZBESCHREIBUNG: SEQ ID NO: 16:
   Glu Leu Thr Gln Ser Pro Lys Phe Met Ser Thr Ser Val Gly Asp Arg
    1               5                  10                  15
   Val Ser Val Thr Cys Lys Ala Ser Gln Asn Val Gly Thr Asn Val Ala
               20                  25                  30
   Trp Tyr Gln Gln Lys Pro Gly Gln Ser Pro Lys Thr Leu Ile Tyr Ser
            35                  40                  45
   Pro Ser Tyr Arg Tyr Ser Gly Val Pro Asp Arg Phe Thr Gly Ser Gly
        50                 55                  60
   Ser Gly Thr Asp Phe Thr Leu Thr Ile Ser Asn Val Gln Ser Val Asp
   65                  70                  75                  80
   Leu Ala Glu Tyr Phe Cys Gln Gln Tyr Asn Ser Tyr Pro Leu Thr Phe
                   85                  90                  95
   Gly Ser Gly Thr Lys Leu Glu Leu
               100

## Beschreibung

Gegenstand der Erfindung sind Aminosäuresequenzen (Peptide), die die Wirkung des Enterotoxins von Clostridium difficile neutralisieren. Außerdem werden die variablen und hypervariablen Regionen dieser Antikörper beschrieben.

Es ist bekannt, daß mit der Einführung der Makrolid-Antibiotika wie Clindamycin vermehrt schwere Darmerkrankungen auftraten, die sich in Form von Durchfällen und im weiteren verlauf als eine mitunter tödlich verlaufende pseudomembranöse Kolitis (PMC) äußerten. Dieser Zusammenhang gab der Erkrankung zunächst den Namen "Clindamycin-assoziierter Durchfall". Heute weiß man, daß nahezu alle in der Klinik eingesetzten Antibiotika und auch Zytostatika das Krankheitsbild der PMC auslösen können.

Klinisch ist die PMC durch eine schwere Diarrhoe gekennzeichnet, die unter anderem aufgrund des starken Elektrolytund Flüssigkeitsverlustes zum Tode führen kann. Je nach der Schwere des Krankheitsbildes treten abdominale Schmerzen, blutige Diarrhoe, Fieber und Leukozytose auf. Bisher erfolgt die Behandlung durch Absetzen des die Krankheit auslösenden Antibiotikums, durch die Gabe von Vancomycin sowie durch Ausgleich des Flüssigkeits- und Elektrolytverlustes.

Das äthiologische Agens der pseudomembranösen Kolitis war lange Zeit völlig unbekannt. Erst 1977 konnte im Stuhl eine bis dahin unbekannte toxische Aktivität nachgewiesen werden, die einen zytotoxischen Effekt auf CHO-Zellen (Chinese hamster ovary carcinoma cells) ausübte. Durch weitere Untersuchungen konnte schließlich nachgewiesen werden, daß die pseudomembranöse Kolitis durch Clostridium difficile und dessen Toxine ausgelöst wird.

Clostridium difficile ist ein obligat anaerobes, grampositives Stäbchenbakterium, das subterminale, ovale Sporen bildet. Biochemisch ist es dadurch charakterisiert, daß es Monosaccharide wie Glukose, N-Acetylglukosamin und N-Acetylneuraminsäure fermentieren kann, nicht aber Mannose, Xylose oder Arabinose. Clostridium difficile ist auch nicht in der Lage, diese Monosaccharide von den Seitenketten des gastrointestinalen Mucins abzuspalten, da ihm Enzyme wie Neuraminidase, β-Galaktosidase oder Sialidase fehlen. Aufgrund dieser biochemischen Unzulänglichkeiten kann sich Clostridium difficile im Darm gesunder Menschen nicht durchsetzen. Bei Störung der Darmflora durch die Gabe von Antibiotika oder Zytostatika kommt es zum Überwuchern der Darmflora durch Clostridium difficile und in der Folge zur PMC.

Clostridium difficile produziert zwei Hauptpathogenitätsfaktoren, das Enterotoxin (Toxin A) und das Zytotoxin (Toxin B). Ihre Gewinnung, Reinigung und Eigenschaften, sowie ihre Verwendung zur Herstellung monoklonaler Antikörper sind in den Europäischen Patentschriften 153 519 und 209 273, den US-Patentschriften 4 879 218 und 5 098 826 sowie der Internationalen Patentanmeldung WO 91/18 293 eingehend beschrieben.

Beim Schutz vor den Folgen einer Infektion mit Clostridium difficile spielen Antikörper offensichtlich eine wichtige Rolle. Bei Patienten, die an PMC erkrankt waren, konnte man Antikörpertiter gegen Toxin A und B feststellen. Hamster entwickeln nach Antibiotikabehandlung und Infektion mit toxinbildenden C. difficile Stämmen das Vollbild der PMC, an der sie versterben. Eine vorherige Immunisierung der Tiere mit den genannten Toxinen schützt sie vor der Erkrankung. Eine Neutralisation der Toxine kann durch Antikörper erzielt werden, die gegen die C-terminale repetitive Ligandendomäne, die zentrale Translokationsdomäne oder aber die N-terminale katalytische Domäne gerichtet sind (zum Domänenaufbau siehe lit [1]). Erstere verhindern die Bindung der Toxine an die Zellrezeptoren, die letzteren blockieren die durch die Toxine vermittelte Glukosylierungsreaktion, während die gegen die zentrale Translokationsdomäne gerichteten Antikörper das Eindringen der Toxine in die Zelle hemmen.

Toxin A neutralisierende Antikörper bieten somit die Möglichkeit zur Therapie und/oder der Prophylaxe vor C.difficile Erkrankungen, wobei sie nicht die Bakterien eliminieren, sondern die Wirkung der gebildeten Toxine blockieren. Auf diese Weise kann die Ausbildung der Krankheitssymptome ursächlich, weil an den Toxinen ansetzend, verhindert werden.

Nimmt man Toxin A als Beispiel, so produziert die Zellinie DSM ACC 2322 einen Antikörper TTC8, der nicht nur an das Toxin A bindet, sondern dessen biologische Wirkung auch zu neutralisieren vermag. Die Bindungsstelle von TTC8 im Toxin A wurde innerhalb der repetitiven Liganden-Domäne lokalisiert. Durch Bindung der Antikörper an die Toxine wird deren Interaktion mit den Zellrezeptoren verhindert. Die Bindungsstelle des mAk TTC8 befindet sich innerhalb der Aminosäuren 2480-2539 des Toxin A, mit der (wahrscheinlichsten) antigenen Sequenz TINGKKYYF. Ein aus dem Patent US 4879218 bekannter mAb PCG-4 bindet an ein durch die Aminosäuren 2098 bis 2141 des Toxin A definiertes Proteinfragment.

Der Beim monoklonalen Antikörper PCG-4 als auch beim monoklonalen Antikörper TCC8 handelt es sich um Maus-Antikörper, die in der Humantherapie nicht eingesetzt werden, als diagnostische Hilfsmittel aber geeignet wären:

Im folgenden werden monoklonale Antikörper, die im Labor des Erfinders generiert worden sind, und ihre Reaktivitäten aufgeführt:

| Gerichtet gegen | Toxin^{a)} | Toxin^{b)} |
|---|---|---|
| Katalytische Domäne^{b)} | 1212 | 2612,2688,3110, 1502, 1115 |
| Translokationsdomäne^{b)} | 2825, 2836, 5288 | 2703, 2740, 2747, 2754, 5288, 2784, 2788 |
| Ligandendomäne^{b)} | 2620, TTC8 | 2912, 2914, 2916, 2926, 2562 2CV |

### Anmerkungen:

a) Die Antikörper erkennen rekombinantes Protein der aufgeführten Regionen im Western Blot.
b) Die Aminosäureposition der Domänen in den Toxinen A und (B) sind: Katalytische Domäne: 1-879 (1-877); Translokationsdomäne: 880-1848 (878-1850); Ligandendomäne: 1849-2681 (1851-2360).

Für alle bisher generierten und beschriebenen Toxinspezifischen monoklonalen Antikörpern gilt, daß sie aus Mäusen gewonnen wurden. Therapeutische oder prophylaktische Anwendung derartiger Antikörper in anderen Spezies außer der Maus (dem Menschen oder anderen Tieren) ist nicht möglich, denn die Antikörper werden als Spezies-fremd erkannt, induzieren eine Immunreaktion, durch die sie inaktiviert werden. Ihre Adaptation auf andere Spezies, insbesondere ihre Humanisierung, war bisher nicht möglich, da ihre variablen und hypervariablen Regionen nicht sequenziert, d.h. nicht definiert waren.

Ziel der Erfindung war es, Peptide verfügbar zu machen, die Toxin-neutralisierende Eigenschaften besitzen, jedoch keinen stark immunogenen Anteil mehr aufweisen, so daß die Peptide sich zum Einsatz in der Human- und Veterinärmedizin eignen. Die Neutralisation der Wirkung der Toxine basiert auf der Bindung der Antikörper, vermittelt durch die Anheftung der variablen Regionen der Antikörper an das jeweilige Toxin. Diese Bindung wird hauptsächlich durch die hypervariablen Regionen (CDR: complementarity determining region) bestimmt. Es stellte sich daher die Aufgabe, die für die Bindung und Neutralisierung der Toxine verantwortlichen Regionen (variable und hypervariable (CDR)) zu identifizieren, und diese derart anzupassen, daß sie keine Immunreaktion mehr auslösen. Solche Peptide können sowohl für die Therapie bereits bestehender Clostridium difficile Erkrankungen als auch zum Schutz vor derartigen Erkrankungen im Bereich der Human- und auch der veterinärmedizin eingesetzt werden.

Die bisher vorliegenden wissenschaftlichen Ergebnisse lassen sich dahingehend zusammenfassen, daß einerseits das Toxin A von Clostridium difficile die volle Symptomatik der pseudomembranösen Kolitis (PMC) auszulösen vermag, andererseits der gegen dieses Toxin gerichtete monoklonale Antikörper TTC8 in vivo (in der Maus) die Wirkung dieses Toxins neutralisieren kann. Der mAk TTC8 ist ein Antikörper der Klasse IgG 2b. Die spezifische Erkennung zwischen dem mAk TTC8 und dem Toxin A von Clostridium difficile wird durch die hypervariablen Regionen der schweren und leichten Ketten vermittelt. Das heißt, daß genau umschriebene Abschnitte des Antikörpers (mit anderen Worten Peptide) für die Antigenerkennung verantwortlich sind. Die Kenntnis der hypervariablen Regionen ermöglicht es, diese Peptide (Strukturen) auf Antikörpersequenzen anderer Spezies zu übertragen, die dann z.B. beim Menschen, in Form humanisierter monoklonaler Antikörper, für Therapie und Prophylaxe der *C. difficile* Erkrankungen geeignet sind und nicht die unerwünschten Begleiterscheinungen von speziesfremden Antikörpern haben, die in der Regel aus der Maus gewonnen werden.

Es stellte sich deshalb die Aufgabe, die Nukleotidsequenzen und die aus ihr abgeleitetin Aminosäuresequenzen der hypervariablen Toxin-neutralisierender monoklonaler Antikörper zu ermitteln, um diese in Form von Teilpeptiden oder aber als humanisierte Antikörper (eingebaut in humane Antikörpergene) für Therapie und Prophylaxe im Menschen einsetzen zu können. Eine derartige Sequenzbestimmung wurde beispielhaft ausgehend von der Zelle DSM 2322 vorgenommen, die den monoklonalen Antikörper TTC8 produziert, der das Enterotoxin A durch Blockade der Ligandendomäne in seiner biologischen Wirkung neutralisiert.

Gelöst und diese Aufgabe dadurch, daß für die Zellinie DSM ACC 2322 mit den Sequenzen SEQ ID No 1-12 und/oder SEQ ID No13-16 die hypervariablen und variablen Peptidsequenzen bestimmt wurden, die die neutralisierende Aktivität des mAb TTC8 ausüben. Die Kenntnis der variablen Regionen neutralisierender Antikörper erlaubt es Peptide zu produzieren, die durch Bindung an das Enterotoxin und/oder das Zytotoxin von Clostridium difficile deren biologische Wirkungen aufheben. Diese Peptidsequenzen, wie die Sequenzen SEQ ID No. 14 und/oder SEQ ID No. 16 des Sequenzprotokolls, können als solche oder aber eingebaut in Immunglobuline zur Therapie der Clostridium difficile Erkrankungen eingesetzt werden. Wenn die Therapie der Menschen angestrebt wird, in humane Immunglobuline, zum Einsatz in der Veterinärmedizin muß der Einbau der Sequenzen in Immunglobulingene der zu behandelnden Spezies erfolgen.
Derivate solcher Peptide können unter Erhalt der biologischen Aktivität durch Aminosäuredeletion, -insertion, -addition oder -austausch, d.h. durch Allelvariation, gewonnen werden. Diese Derivate können wie die ursprünglichen Peptide durch Bindung an das Enterotoxin und/oder das Zytotoxin von Clostridium difficile deren biologische Wirkungen aufheben und somit zu Therapie und Prophylaxe der Clostridium difficile Erkrankungen eingesetzt werden.

Die nachstehend genannten hypervariablen Regionen (CDRs = complenentarity determining regions) wurden in dem mAk TTC8 bestimmt, der aus der bei der Deutschen Sammlung von Mikroorganismen und Zellkulturen GmbH (DSMZ) hinterlegten Hybridoma-Zellinie DSM ACC 2322 isoliert worden ist. Sie weisen folgende Aminosäuresequenzen auf:
CDR der schweren Kette:
CDR der leichten Kette :

Zur Bestimmung dieser Sequenzen wurde wie folgt vorgegangen: Zunächst wurde die Gesamt-RNA der Hybridomazellen, die den mAk TTC8 bilden, nach einem an sich bekannten Verfahren präpariert. Aus der Gesamt-RNA wurde dann in einem zweiten Schritt die mRNA abgetrennt. Dies erfolgte mit Hilfe von magnetischen Polystyrol-Kugeln (Beads), an die Oligo(dT)₂₅-Ketten kovalent gebunden sind.

Aus der gereinigten mRNA wurde danach cDNA synthetisiert und mit Hilfe der Polymerase-Kettenreaktion (PCR) das VH- und das VL-Gen des mAk TTC8 herausamplifiziert, die für die leichte (VL) bzw schwere Kette (VH) des Antikörpers TTC8 kodieren. Hierbei war es von entscheidender Bedeutung, daß Primer ausgewählt wurden, die eine geeignete Restriktionsschnittstelle aufwiesen, um die anschließende Klonierung zu erleichtern.

Danach wurden die so gewonnenen VH- und VL-Gene des mAk TTC8 in dem Vektor pUC 19 kloniert und dann sequenziert. Dabei wurden die Nukleotid- (SEQ ID No. 13 und 15) und aus ihr abgeleitet die Aminosäuresequenzen entsprechend SEQ ID No. 14 und SEQ ID No. 16 gefunden.

Anhand eines Vergleiches mit dem Keimbahngen V 102 lassen sich hieraus die hypervariablen Bereiche identifizieren. Aus dem Gen der schweren Kette (SEQ ID No. 13) ist zu erkennen, daß die CDR-1 der schweren Kette 5 Aminosäuren umfaßt und in Position 30 der Sequenz beginnt. Die CDR-2 der schweren Kette beginnt in Position 49 und umfaßt 17 Aminosäuren. Die hypervariable Region CDR-3 beginnt in Position 98 und umfaßt 11 Aminosäuren.

Insgesamt weist das VH-Gen des mAk TTC8, 36 Punktmutationen im vergleich zum Keimbahngen V 102 auf.. Drei der Mutationen liegen im Bindungsbereich des PCR-Primers und können deshalb durch die Sequenz des Primers bedingt sein. Fast alle Mutationen in den hypervariablen Regionen führen zu einer Veränderung der Aminosäuresequenz, während annähernd die Hälfte aller Mutationen in den Framework-Regionen stumm sind.

Im Gen für die leichte Kette (SEQ ID No. 15) konnten die hypervariablen Regionen wie folgt festgelegt werden: Die CDR-4 beginnt in Position 22 und umfaßt 11 Aminosäuren. Die CDR-5 umfaßt 7 Aminosäuren und beginnt in Position 48. Die CDR-6-Region beginnt an der Position 87 und umfaßt 9 Aminosäuren. Das VL-Gen des mAk TTC8 weist im vergleich zum mAk A23 nur 9 Mutationen auf. Davon liegen 4 im Bindungsbereich des PCR-Primers. Von den übrigen fünf Unterschieden in der Nukleotidsequenz sind zwei Mutationen auf der Proteinebene stumm. Von den Mutationen, die zu einer Veränderung der Aminosäuresequenz führen, liegt eine in der CDR-4, die übrigen beiden in den Framework-Regionen 2 und 3.

Die therapeutische Verwendung eines von der Maus abgeleiteten monoklonalen Antikörpers ruft im Menschen eine Immunreaktion hervor. Um dieses Problem zu überwinden, kann ein derartiger Antikörper auf die zu behandelnde Spezies (hier der Mensch) angepaßt, d.h. humanisiert werden. Dafür gibt es mehrere Verfahren, die darauf hinauslaufen, den immunogenen Anteil des murinen monoklonalen Antikörpers durch entsprechende Anteile eines humanen Antikörpers zu ersetzen. Sie sind beispielsweise aus den europäischen Patentanmeldungen 184 187, 171 496 und 173 494 bekannt.

Die nach einem derartigen Verfahren gewonnenen humanisierten monoklonalen Antikörper sind für die therapeutische Anwendung am Menschen wesentlich geeigneter als die aus der Maus gewonnenen Antikörper.

Diese Methoden zur Humanisierung von Antikörpern lassen sich auch auf den mAk TTC8 und andere Toxin A und neutralisierende monoklonale Antikörper anwenden und ergeben einen humanisierten monoklonalen Antikörper, der eine Chimäre darstellt aus den hypervariablen Regionen (z.B. für TTC8: SEQ ID No. 1 - 12) des neutralisierenden mAk TTC8 insertiert in die Framework-Region eines humanen Immunglobulins. Letzteres kann vom Subtyp IgG (bevorzugt für die parenterale Applikation) oder vom Subtyp IgA (bevorzugt für die perorale Applikation) sein. wie für den Antikörper TTC8 (aus der Zellinie DSM ACC 2322) beschrieben, können entsprechend auch die hypervariablen Regionen anderer gegen Clostridium difficile gerichteter monoklonaler Antikörper kloniert, sequenziert und zur Herstellung humanisierter Antikörper eingesetzt werden.

Gegenstand der Erfindung sind auch humanisierte monoklonale Antikörper, bei denen innerhalb der variablen und/oder konstanten Regionen der leichten und/oder schweren Ketten des humanen Immunglobulins die Aminosäuresequenz durch Allelvariationen modifiziert ist, so lange die Bindungsfähigkeit an das Toxin A und/oder an das Toxin B von Clostridium difficile erhalten bleibt.

Alle für die Herstellung der monoklonalen Antikörper erforderlichen biologischen "Bausteine" wie Zellinien, Plasmide, Promotoren, Resistenzmarker, Replikationsursprünge und andere Bruchstücke von Vektoren sind, soweit sie nicht wie hier beschrieben, hinterlegt sind, im Handel erhältlich oder allgemein verfügbar. Falls nicht anders angegeben, werden sie nur als Beispiele verwendet und sind nicht für die Durchführung der Erfindung entscheidend, sondern können auch durch andere geeignete biologische "Bausteine" ersetzt werden. Bakterielle Wirtszellen werden vorzugsweise zur Amplifizierung der erfindungsgemäß genannten DNA-Sequenzen verwendet. Beispiele hierfür sind E. coli oder Bacillus spec.

Die Herstellung der humanisierten Antikörper kann in eukaryontischen Zellen wie Hefezellen, Pilzen oder z.B. CHO (Chinese hamster ovary cells) erfolgen. Die Produktion in Pflanzen kann in monokotylen oder dikotylen Pflanzen erfolgen.

Beispielhaft wird im folgenden die Herstellung von Antikörpern in Pflanzen beschrieben. Die Herstellung in anderen Organismen erfolgt analog.

Die Herstellung bestimmter Antikörper in Pflanzen ist bereits aus [2] und der Internationalen Patentanmeldung WO 91/06320 bekannt. Für die Herstellung des monoklonalen Antikörpers in transgenen Pflanzen werden die Gene für den vollständigen monoklonalen Antikörper oder für seine rekombinanten Derivate bzw. das Gen für einen einkettigen Antikörper unter Kontrolle eines oder zweier in Pflanzen aktiver Promotoren in einen Pflanzentransformationsvektor kloniert.

Der fertige Transformationsvektor enthält alle in die Pflanzen zu transferierenden DNA-Sequenzen. Diese werden aus dem Vektor in die Pflanzenzelle transferiert. Alternativ können die Gene für die leichte und die schwere Kette auch getrennt in zwei Pflanzentransformationsvektoren eingebaut und getrennt in Pflanzenzellen transferiert werden, um sie erst später zum fertigen Antikörper zusammenzufügen. Die Transformation von Pflanzen kann mit allen geeigneten Verfahren, z.B. mit Hilfe von Agrobacterium tumefaciens oder durch direkten Gentransfer oder mit Hilfe der Partikelkanone durchgeführt werden.

Die Produktion der Antikörper kann zielgerichtet in einzelne Kompartimente der Zelle gesteuert werden. Bei Entfernung der für das Signalpeptid kodierenden Sequenz werden die Antikörper zytoplasmatisch exprimiert. Für eine Hochexpression der Antikörper wird an das 5'-Ende des Gens eine DNA-Sequenz angekoppelt oder die natürlich vorhandene belassen, die für ein Signalpeptid zum Eintransport in das endoplasmatische Retikulum kodiert. Um bestimmte Lokalisierungen in definierten Zellkompartimenten zu erreichen, können DNA-Sequenzen, die für die dafür verantwortlichen Peptidsequenzen kodieren, an oder in das Gen fusioniert werden. Durch Integration einer KDEL-Sequenz kann z.B. eine Retention im endoplasmatischen Retikulum erreicht werden.

Der Einbau der Antikörper-Gene in der transgenen Pflanze wird durch geeigneten Restriktionsverdau der isolierten genomischen Pflanzen-DNA und nachfolgende Southern Hybridisierung analysiert und nachgewiesen. Die Transkription der Gene kann im Northern Blot nachgewiesen werden. Der biosynthetisierte Antikörper wird z.B. mit Hilfe eines spezifischen sekundären oder eines gegen die konstante Region oder gegen eine speziell zu diesem Zweck eingeführte (sogenannte Tag-) Sequenz gerichteten polyklonalen oder monoklonalen Antikörpers in Pflanzenextrakten nachgewiesen.

Als Produktionspflanzen eignen sich sowohl monokotyle Pflanzen wie z.B. Gerste und Weizen als auch dikotyle Pflanzen wie z.B. die Kartoffel, Raps, die Mohrrübe oder die Erbse. Die Expression des Antikörpers kann in verschiedenen Organen der Pflanzen, z.B. in Blättern, in Samen, Knollen oder anderen Geweben erfolgen.

Die Aufreinigung des in der Pflanze exprimierten Antikörpers erfolgt z.B. mit chromatographischen Methoden, die üblicherweise auch für die Aufreinigung von Antikörpern aus Hybridoma-Zellinien benutzt werden. Weiterhin können rekombinante Antikörper, die eine Tag-Sequenz enthalten, auch mittels speziell dafür etablierter Affinitätschromatographie-Verfahren, z.B. der Metallchelatchromatographie, gereinigt werden.

Die humanisierten monoklonalen Antikörper können dem menschlichen Patienten zur Therapie und Prophylaxe von Erkrankungen, die durch Clostridium difficile hervorgerufen werden, nach an sich bekannten Verfahren verabreicht werden. Im allgemeinen werden die Antikörper oder Antikörperfragmente parenteral, vorzugsweise jedoch per os verabreicht. Als spezielle "galenische Zubereitung" kommt die direkte perorale Einnahme von Pflanzen, die die Antikörper enthalten, in Form von Rohkost in Betracht. Die geeignete Dosierung der Antikörper ist auf den jeweiligen Patienten anzupassen und ist z.B. abhängig von seinem Körpergewicht, seinem Alter und seinem Krankheitszustand. Sie wird von dem erfahrenen Arzt festgelegt und liegt üblicherweise zwischen 0,1 mg/kg und 70 mg/kg, die einmal oder mehrmals täglich über einen Zeitraum von mehreren Tagen verabreicht wird.

### Beispiele

### Beispiel 1: Gewinnung der Gesamt-RNA der Hybridomazellen, die den mAk TTC8 bilden (DSM ACC 2322)

Für die Reinigung der RNA durch CsCl-Gradienten werden die Zellen initial durch Guanidinium-Thiocyanat-Puffer aufgeschlossen und anschließend mechanisch mit Hilfe eines Ultrathorax vollständig zerstört. Zelltrümmer werden abzentrifugiert und die Lösung auf ein vorbereitetes CsCl-Polster (5.7 M) aufgegeben. Bei der folgenden Zentrifugation pelletiert die RNA auf den Boden des Röhrchens. Dieser Boden wird mit einem heißen Skalpell abgetrennt und das Pellet durch Zugabe von H₂O gelöst. Aus der Lösung wird die RNA dann durch Fällung weiter angereinigt. Am Ende wird sie in 100 µl H₂O aufgenommen. Die Konzentration der so präparierten RNA liegt in der Regel bei 1.5-3 µg/µl.

### Beispiel 2: Isolierung der mRNA

Zur Reinigung der mRNA von den anderen RNA-Spezies wurde sie an Oligo(dT)₂₅-Beads nach dem von Dynal beschriebenen Verfahren hybridisiert. Die Bindekapazität der Beads beträgt 2µg RNA pro mg Beads. Der Anteil der mRNA an der Gesamt-RNA beträgt etwa 1-5%, so daß 1mg Beads mit 80µg Gesamt-RNA beschickt wurde. Die Reinigung erfolgte nach dem Protokoll der Firma Dynal mit folgenden Abweichungen. Die gebundenen mRNA wurde zweimal mit einem Puffer der Zusammensetzung 10mM Tris/HCl (pH 7.5), 0,15 mM LiCl, 1mM EDTA, 1% SDS und danach zweimal mit einem abgewandelten Puffer geringerer Salzkonzentration [5mM Tris/HCL (pH 7.5), 75mM LiCl, 0.5mM EDTA] gewaschen. Alle weiteren Schritte entsprachen wieder den Angaben von Dynal. Der Zusatz von SDS und der zusätzliche Waschschritt bei niederer Salzkonzentration (höherer Stringenz) verbesserte die Reinheit der mRNA wesentlich.

### Beispiel 3: Synthese der cDNA

Aus der gereinigten mRNA wurde unter Einsatz von MMLV-reverse transcriptase (Moloney Murine Leukemia Virus) cDNA synthetisiert. Dabei wurde der Oligo(dT)₁₂₋₁₈ Primer in 2x Überschuß eingesetzt, um den Anteil der hybridisierten mRNA-Oligo(dT) Moleküle hoch zu halten. In der Reaktion war die Konzentration der Nukleotidtriphosphate größer als 1mM. Einem vorzeitigen erhöhten Abbau der RNA in der Lösung wurde durch Zusatz von humanem und plazentalem Ribonuklease-Inhibitor entgegengewirkt. Die so gewonnene ssDNA wurde in die PCR-Reaktionen zur Amplifikation der variablen Regionen des mAb TTC8 eingesetzt.

### Beispiel 4: Die Polymerase-Kettenreaktion zur Amplifikation der cDNA

Die Amplifikation der cDNA wurde entsprechend dem aus der Europäischen Patentschrift 0 388 914 bekannten Verfahren durchgeführt.

Anhand einer Reihe von Publikationen wurden eigene Primer zur PCR-Amplifikation der variablen Regionen von monoklonalen Antikörpern der Species maus entworfen. Diese sind:
Schwere Kette: (unterstrichen eine eingeführte *Sal*I Schnittstelle)
Leichte Kette: (unterstrichen eine eingeführte *Sac*I Schnittstelle)

Die optimale Primer-Konzentration für die Gewinnung der variablen Regionen betrug 0.5 µM, die dNTPs wurden in einer Endkonzentration von 400µM eingesetzt.

Als zweiter wesentlicher Parameter wurden die PCR-Temperaturen festgelegt. Für die VH-Amplifikation war die Reaktionsabfolge letztlich: Denaturierung 1 min 92°C; Annealing 1.5 min 50°C; Elongation 3 min 72°C, bei 30 Zyklen. Für die VL-Amplifikation war die Reaktionsabfolge letztlich: Denaturierung 1 min 92°C; Annealing 1.5 min 60°C; Elongation 3 min 72°C, bei 30 Zyklen.

### Beispiel 5: Die Klonierung des VH- und des VL-Gens des mAk TTC8

Zur Klonierung wurden die PCR-Produkte mit *Sal*I (VH) oder *Sac*I (VL) geschnitten und über ein Agarosegel gereinigt. Die klonierung erfolgte in pUC19 in die homologen Schnittstellen.

Als Zielklone wurden die Konstrukte pVHT8 und pVLT8.10 durch Kontrollverdaus identifiziert. Sie enthalten die VH- bzw VL-Segmente in positiver Orientierung in Bezug auf den pLac Promotor des pUC19.

### Beispiel 6: Die Sequenzierung der variablen Domäne des mAk TTC8

Das Insert des pVHT8 hat eine Größe von 341 bp, das des Klons pVLT8.10 eines von 312 bp. Beide Klone besitzen je ein offenes Leseraster, das sich über das gesamte Insert erstreckt. Die Sequenzen der PCR-Primer und die Schnittstellen ließen sich gut identifizieren.

Durch Sequenzvergleich ergibt sich die Zuordnung des VH-Segments zur Multigenfamilie J558. Der höchste Grad der Homologie ergab sich zur Keimbahnsequenz V102.

Das Gen für die VL-Region des mAb TTC8 hat eine 94%ige Homologie zu einer Sequenz eines Antikörpers, der zum Keimbahngen vk19d gerechnet wird. Demnach ist auch die vL-Region des TTC8 dieser Genfamilie zuzuordnen.

### Beispiel 7: Die Herstellung eines humanisierten monoklona len Antikörpers

Um bei der Applikation von Antikörpern anderer Spezies (im folgenden als nonhuman bezeichnet) eine Reaktion des menschlichen Immunsystems zu verhindern, müssen die immunogenen Regionen der mAk durch homologe humane Sequenzen ersetzt werden. Hierzu gibt es im wesentlichen zwei Möglichkeiten:
a) die Bildung von chimären Maus/Mensch-Antikörpern, wobei die variable, antigen-determinierende Region des chimären Antikörpers dem nonhumanem Antikörper, die konstante Region einem humanen Antikörper entstammt
b) die vollständige Humanisierung des mAks. Dabei werden auch die Framework-Bereiche in der variablen Region des mAks durch entsprechende humane Sequenzen ersetzt und dann nur noch die CDR-Regionen im Original oder als Allelvariate beibehalten.

Für die praktische Durchführung kann auf eine Reihe von Methoden zurückgegriffen werden, die gut publiziert und Fachleuten aus dem Gebiet wohlbekannt sind.

zu a) Chimäre Antikörper werden dadurch erzeugt, daß in einem geeigneten Vektorsystem die Sequenzen für die konstanten Regionen eines humanen Antikörpers an die Sequenzen für die variable Region des nonhumanen mAk gekoppelt werden. Es kann sowohl ein kompletter chimärer Antikörper hergestellt werden oder auch nur ein Teil des chimären Antikörpers, ein sogn. Fab-Fragment [3]. Die Herstellung chimärer Antikörper hat den Vorteil, daß sie methodisch relativ problemlos ist. Die so erzeugten Antikörper weisen, aufgrund der verbleibenden Maus-Anteile im Protein, eine leicht erhöhte Immunogenität auf, die aber bei der peroralen Applikation nur eine untergeordnete Rolle spielen.

zu b) Für die vollständige Humanisierung von nonhumanen mAks wird zunächst durch Sequenz-Vergleich und 3D-Modelling ein humaner Antikörper identifiziert, dessen Struktur in etwa dem des ursprünglichen Antikörpers entspricht. Anschließend wird durch die Verwendung von fünf Oligonukleotid-Primern, die in der Sequenz der variablen Region des humanen Antikörpers (VL oder VH) binden, sowie von drei Oligonukleotiden, die die Sequenzen der CDR-Regionen des Maus-Antikörpers beinhalten, mit Hilfe von aufeinander aufbauenden PCR-Amplifikationen ein vollständiges, humanisiertes Gen für den variablen Anteil des Antikörpers erzeugt [4]. Ein zweiter modifizierter Ansatz besteht darin, das Gen aus mehreren synthetischen, überlappenden Oligonukleotiden, die für die Zielsequenz kodieren, zusammenzusetzen [zu Strategie und Durchführung vergl.4]. Alternativ besteht die Möglichkeit nach der Identifizierung eines homologen humanen Antikörpers nur die Aminosäuren, in denen sich die Framework-Regionen beider Ak unterscheiden, durch gezielte Mutagenese anzugleichen [5]. So modifizierte Antikörper können einer Veränderung ihrer Spezifität unterliegen, die durch Rückmutationen wieder ausgeglichen werden [5]. Die vollständige Humanisierung von Antikörpern auf dem zuletzt genannten Weg erfordert einen relativ hohen methodischen Aufwand, allerdings erzeugen solche Antikörper praktisch keine Immunantwort im Menschen [6].

### Literaturzusammenstellung

[1] Eichel-Streiber, C.v., P. Boquet, M. Sauerborn und M. Thelestam. 1996. Large clostridial cytotoxins - a family of glycosyltransferases modifying small GTP-binding proteins. Trends in Microbiology, 14: 375-382.
[2] Düring, K. (1988) "Wundinduzierte Expression und Sekretion von T4 Lysozym und Monoklonalen Antikörpern in Nicotiana tabacum." Ph.D. Thesis, Universität Köln
[3] Skerra A. (1994). A general vector, pASK 84, for cloning, bacterial production and single step purification of antibody Fab fragments. Gene 141:79-84.
[4] Sato K., M. Tsuchiya, J. Saldanha, Y. Koishihara, Y. Ohsugi, T. Kishimoto und M.M. Bendig (1994). Humanization of a mouse anti-human Interleukin-6 receptor antibody comparing two methods for selecting human framework regions. Mol. Immun. 31:371-381.
[5] Benhar I., E.A. Padlan, S.H. Lee, B. Lee und I. Pastan (1994). Rapid humanization of the Fv of monoclonal antibody B3 by using framework exchange of the recombinant immunotoxin B3(Fv)-PE38. Proc. Natl. Acad. Sci. USA 91:12051-12055.
[6] Stephens S., S. Emtage, O. Vetterlein, L. Chaplin, C. Bebbington, A. Nesbitt. M. Sopwith, D. Athwal, C. Nowak und M. Bodmer (1995). Comprehensive pharmacokinetics of a humanized antibody and analysis of residual anti-idiotypic responses. Immunology 85: 668-674. reaction to generate humanised monoclonal antibodies", GENE, Bd. 101. Nr. 2, 1. Januar 1991, Seiten 297-302.
[8] Lyerly et al.: "Vaccination against lethal Clostridium difficile Enterocolitis with a non-toxic recombinant peptide of toxin A", CURRENT MICROBIOLOGY, Bd. 21, Juli 1990, Seiten 29-32.
[9] Corthier G. et al.: "Protection against experimental pseudomembranous colitis in gnotobiotic mice by use of monoclonal antibodies against Clostridium difficile toxin A", INFECTION AND IMMUNITY, Bd. 59, Nr. 3, März 1991, Seiten 1192-1195.

## Patentansprüche

1. Aminosäuresequenz, **dadurch gekennzeichnet, dass** sie vollständig oder anteilig Aminosäuresequenzen ausgewählt aus SEQ. ID. No. 2, SEQ. ID. No. 4, SEQ. ID. No. 6, SEQ. ID. No. 8, SEQ. ID. No. 10 oder SEQ. ID. No. 12 des Sequenzprotokolls enthält und das Clostridium difficile Toxin A bindet.

2. DNA-Sequenz, **dadurch gekennzeichnet, dass** sie für eine oder mehrere der Aminosäuresequenzen von Anspruch 1 codiert.

3. DNA-Sequenz nach Anspruch 2, **dadurch gekennzeichnet, dass** sie eine oder mehrere Nucleotidsequenzen ausgewählt aus SEQ. ID. No. 1, SEQ. ID. No. 3, SEQ. ID. No. 5, SEQ. ID. No. 7, SEQ. ID. No. 9 oder SEQ. ID. No. 11 des Sequenzprotokolls enthält.

4. Aminosäuresequenz, **dadurch gekennzeichnet, dass** sie eine Sequenz ausgewählt aus SEQ. ID. No. 14 und SEQ. ID. No. 16 des Sequenzprotokolls enthält oder aus ihr besteht.

5. DNA-Sequenz, **dadurch gekennzeichnet, dass** sie eine Nucleotid-Sequenz ausgewählt aus SEQ. ID. No. 13 und SEQ. ID. No. 15 des Sequenzprotokolls enthält oder aus ihr besteht.

6. Aminosäuresequenz nach den Ansprüchen 1 oder 4, **dadurch gekennzeichnet, dass** sie eine oder mehrere durch Allelvariationen modifizierte Derivate der Aminosäuresequenzen enthält, die eine vergleichbare biologische Wirkung aufweisen wie die Aminosäuresequenzen von Anspruch 1.

7. DNA-Sequenz, **dadurch gekennzeichnet, dass** sie für eine oder mehrere der Aminosäuresequenzen von Anspruch 6 codiert.

8. Hybridoma-Zelllinie DSM AC 2322.

## Claims

1. Amino acid sequence, **characterized in that** it completely or partly contains amino acid sequences, selected from SEQ ID NO 2, SEQ ID NO 4, SEQ ID NO 6, SEQ ID NO 8, SEQ ID NO 10 or SEQ ID NO 12 of the sequence protocol, and that it binds the Toxin A from *Clostridium difficile*.

2. DNA Sequence, **characterized in that** it encodes one or more of the amino acid sequences of claim 1.

3. DNA Sequence according to claim 2, **characterized in that** it contains one or more nucleotide sequences selected from SEQ ID NO 1, SEQ ID NO 3, SEQ ID NO 5, SEQ ID NO 7, SEQ ID NO 9 or SEQ ID NO 11 of the sequence protocol.

4. Amino acid sequence, **characterized in that** it contains or that it consists of a sequence selected from SEQ ID NO 14 and SEQ ID NO 16 of the sequence protocol.

5. DNA sequence, **characterized in that** it contains or that it consists of a nucleotide sequence selected from SEQ ID NO 13 and SEQ ID NO 15 of the sequence protocol

6. Amino acid sequence according to claims 1 or 4, **characterized in that** it contains one or more modified derivatives of the amino acids being obtained by allelic variation, said derivatives have a biological effect comparable to that of the amino acid sequences of claim 1.

7. DNA sequence, **characterized in that** it encodes for one or more amino acid sequences of claim 6.

8. Hybridoma cell line DSM ACC 2322.

## Revendications

1. Séquence d'acides aminés, **caractérisée en ce qu**'elle est constituée de séquences d'acides aminés complètes ou de parties de celles-ci choisies parmi les séquences SEQ. ID. N° 2, SEQ. ID. N° 4, SEQ. ID. N° 6, SEQ. ID. N° 8, SEQ. ID. N° 10 ou SEQ. ID. N° 12 du protocole de séquences, qu'elle lie la toxine clostridium difficile.

2. Séquence d'ADN, **caractérisée en ce qu**'elle code une ou plusieurs séquences d'acides aminés selon la revendication 1.

3. Séquence d'ADN selon la revendication 2, **caractérisée en ce qu**'elle comprend une ou plusieurs séquences de nucléotides choisies parmi les séquences SEQ. ID. N° 1, SEQ. ID. N° 3, SEQ. ID. N° 5, SEQ. ID. N° 7, SEQ. ID. N° 9 ou SEQ. ID. N° 11 du protocole de séquences.

4. Séquence d'acides aminés, **caractérisée en ce qu'**elle comprend une séquence choisie parmi les séquences SEQ. ID. N° 14 et SEQ. ID. N° 16 du protocole de séquences, ou est constituée par celle-ci.

5. Séquence d'ADN, **caractérisée en ce qu**'elle comprend une séquence de nucléotides choisie parmi SEQ. ID. N° 13 et SEQ. ID. N° 15 du protocole de séquences, ou est constituée par celle-ci.

6. Séquence d'acides aminés selon les revendications 1 ou 4, **caractérisé en ce qu**'elle comprend un ou plusieurs dérivés des séquences acides aminés modifiés par variation allèle et qui présentent des effets biologiques comparables à ceux des séquences d'acides aminés de la revendication 1.

7. Séquence d'ADN, **caractérisée en ce qu**'elle code une ou plusieurs séquences d'acides aminés de la revendication 6.

8. Lignée cellulaire hybridome DSM AC 2322.
